# EUROPEAN PATENT APPLICATION

(11) **EP 2 420 829 A2**
(43) Date of publication of application: **22.02.2012**
(21) Application number: 10764694.5
(22) Date of filing: 19.04.2010
(51) Int. Cl.: G01N 33/49, G01N 33/50

(54) **DISEASE DIAGNOSING BIOSENSOR CAPABLE OF PROMPTLY SEPARATING BLOOD CELLS**

(30) Priority: 17.04.2009 KR 20090033729
(71) Applicant: Digital Optics Co., Ltd., Kyonggi-do 462-120 (KR)
(72) Inventor: MOK, Rak Sun, Busan 609-322 (KR); KIM, Ki Tae, Seongnam-si Gyeonggi-do 463-836 (KR)
(74) Representative: Behnisch, Werner
(86) International application number: PCT/KR2010/002406
(87) International publication number: WO 2010/120155

(57) **Abstract**

The present invention relates to a disease diagnosing biosensor capable of promptly separating blood cells, and more specifically, to a disease diagnosing biosensor capable of promptly separating blood cells, in which a disease can be analyzed further accurately by adding a blood cell separating pad on a front or rear side of a capillary tube and separating the blood cells and plasma within 1 or 2 seconds owing to distribution of hydrophilic and hydrophobic segments.

The entire configuration of the biosensor according to the present invention includes a base film; electrodes arranged on a top surface of the base film; a space film covering a top surface of the electrodes; a blood cell separating pad connected to a rear side of the space film; a capillary tube added on a rear top surface of the space film; and a cover film covered on a top surface of the capillary tube and the blood cell separating pad.

## Description

### TECHNICAL FIELD

The present invention relates to a disease diagnosing biosensor capable of promptly separating blood cells, and more specifically, to a disease diagnosing biosensor capable of promptly separating blood cells, in which a disease can be analyzed further accurately by adding a blood cell separating pad on a front or rear side of a capillary tube and separating the blood cells and plasma within 1 or 2 seconds owing to distribution of hydrophilic and hydrophobic segments.

### BACKGROUND ART

When a biosensor is used to diagnose various kinds of diseases (diabetes, hyperlipidemia and the like) using blood, a method of using plasma or blood cells extracted from the blood, rather than using whole blood, is highly accurate.

Since accuracy and reproducibility of a result are extremely low depending on a distribution network of the blood cells (hematocrit) when the whole blood is used, serums and plasma are extracted from the blood and used as a specimen in testing the blood at a general hospital.

Conventional biosensors adopt a method of performing a blood test using serums or plasma extracted through a preprocess using a centrifuge or a separately provided tool, or diagnosing a disease using whole blood, and approaching values of the measured plasma or serums using a correction equation based on values obtained from the test or diagnosis.

Even in this case, errors may occur with respect to real values depending on the method or extent of the correction, and accuracy and reproducibility of the result are lowered due to these phenomena.

### DISCLOSURE OF INVENTION

### TECHNICAL PROBLEM

Therefore, the present invention has been made in view of the above problems, and it is an object of the present invention to provide a disease diagnosing biosensor capable of promptly separating blood cells, in which a blood cell separating pad is attached on a front or rear side of a capillary tube, and thus plasma and the blood cells may be promptly extracted.

Another object of the present invention is to provide a disease diagnosing biosensor capable of promptly separating blood cells, in which the blood cells and plasma contained in blood are promptly separated, and various kinds of diseases are analyzed using the blood cells and plasma, thereby diagnosing the diseases further accurately.

### TECHNICAL SOLUTION

To accomplish the above object, according to one aspect of the present invention, there is provided a disease diagnosing biosensor capable of promptly separating blood cells, the biosensor comprising: a base film; electrodes arranged on a top surface of the base film; a space film covering a top surface of the electrodes; a blood cell separating pad connected to a rear side of the space film; a capillary tube added on a rear top surface of the space film; and a cover film covered on a top surface of the capillary tube and the blood cell separating pad.

A hydrophilic segment having a length of 1 to 6mm is formed on a bottom surface of the blood cell separating pad.

In addition, the blood cell separating pad is added to either a front side or a rear side of the capillary tube.

Meanwhile, the blood cell separating pad is manufactured using any one of cellulose, fusion 5, glass fiber, cotton, and wool.

In addition, the blood cell separating pad is manufactured to have a width of 1 to 10mm.

Hereinafter, the entire configuration of the present invention and unique effects and the like obtained from the present invention will be described in detail with reference to the accompanying drawings.

### ADVANTAGEOUS EFFECTS

According to the present invention configured as described above, although blood is used as a specimen, since a variance related to the amount of blood cells (hematocrit) is removed by using plasma in diagnosing diseases, a stable diagnosing system that do not need to correct measured values can be constructed.

Furthermore, the biosensor configured as described above is a useful invention which allows blood tests to be performed further accurately compared with a field test by expanding utilization of the biosensor to a diabetes test, a hyperlipidemia test, a biochemical test, and the like.

### BRIEF DESCRIPTION OF THE DRAWINGS

FIG. 1(a) is a plan view showing the configuration of a disease diagnosing biosensor according to an embodiment of the present invention.

FIG. 1(b) is a vertical cross-sectional view showing the configuration of a disease diagnosing biosensor according to an embodiment of the present invention.

FIG. 2 (a) is a plan view showing the configuration of a disease diagnosing biosensor according to another embodiment of the present invention.

FIG. 2(b) is a vertical cross-sectional view showing the configuration of a disease diagnosing biosensor according to another embodiment of the present invention.

FIGs. 3 (a) to 3(d) are views showing the process of manufacturing a disease diagnosing biosensor of the present invention.

<Descriptions of symbols>

10: Base film 15: Electrode

20: Space film 25: Capillary tube

30: Blood cell separating pad

### BEST MODE FOR CARRYING OUT THE INVENTION

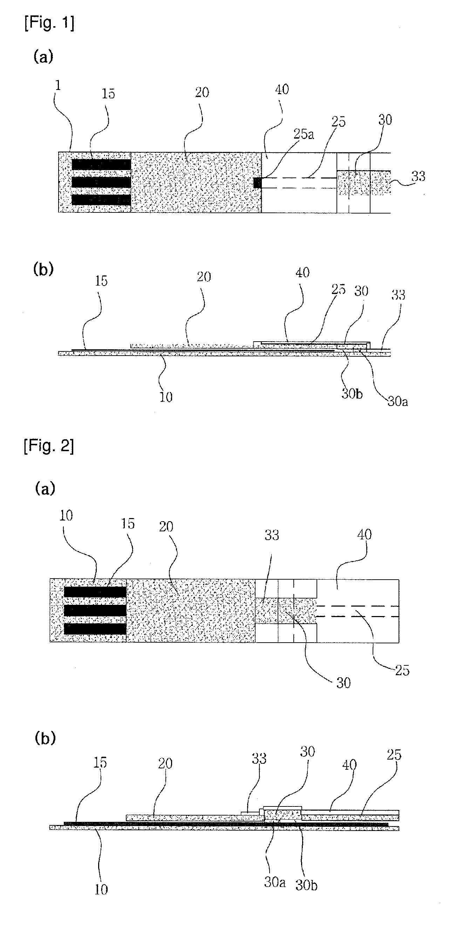

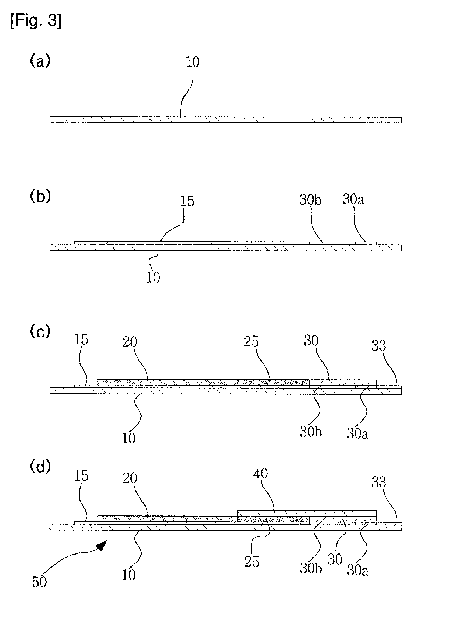

FIGs. 1 to 3 are front views showing the configuration of a biosensor according to the present invention. As shown in the figures, the biosensor of the present invention includes a base film 10, electrodes 15 arranged on the top surface of the base film 10, a space film 20 covering the top surface of the electrodes 15, a blood cell separating pad 30 (← 25) surface-connected to the rear side of the space film 20, a capillary tube 25 (← 30) added on the rear top surface of the space film 20, and a cover film 40 covered on the top surface of the blood cell separating pad 30 (← 35) and the capillary tube 25 (← 30).

The base film 10 is formed using a synthetic resin such as PET, PP, PVC or the like, and two or three electrodes 15 are arranged on the top surface of the base film 10 at regular intervals.

The electrodes 15 are manufactured using a conductive mixture of carbon, gold, platinum, and silver.

In addition, the space film 20 covered on the top surface of the electrodes 15 is formed using a synthetic resin such as PET, PP, PVC or the like.

In addition, the capillary tube 25 having a narrow width is added at the center of the rear side of the space film 20, and a vent 25a is added outside the capillary tube 25.

The blood cell separating pad 30 contacted with the capillary tube 25 is added outside the capillary tube 25.

The blood cell separating pad 30 is manufactured using any one of cellulose, fusion 5, glass fiber, cotton, and wool, and its functions and effects are almost the same.

In addition, the function of the blood cell separating pad 30 may be efficiently enhanced since the blood cell separating pad 30 is manufactured to have a width of 1 to 10mm.

A hydrophobic segment 30a and a hydrophilic segment 30b (← 30a) of a certain width are formed on the bottom surface of the blood cell separating pad 30. The hydrophobic segment 30a is added with a double-sided tape, and the hydrophilic segment 30b is a space unit where nothing is added thereto.

Meanwhile, the present invention is completed by bonding the cover film 40 formed using a resin such as PET, PP, PVC or the like on the top surface of the capillary tube 25 and the blood cell separating pad 30.

In the present invention having a structure described above, if blood is provided to a blood injection hole 33, plasma and serums are separated due to difference of resistance while the blood cells pass through the hydrophobic segment 30a, and the separated plasma passes through the hydrophilic segment 30b and moves to the capillary tube 25 (← 30) faster than the blood cells.

The plasma moved as described above is promptly absorbed into the capillary tube 25 (← 30) in 1 or 2 seconds owing to absorption force of the capillary tube phenomenon.

The plasma transferred as described above is used to diagnose various kinds of diseases using a conventional electrochemical or liquid color developing method.

The process of manufacturing a biosensor of the present invention configured as such will be described below. First, two or three electrodes 15 are arranged at regular intervals on the top surface of the base film 10 cut into a piece having a certain width and length.

The hydrophobic segment 30a is formed at one side of the electrodes 15 by attaching a double-sided tape of a certain width at regular intervals in the vertical direction, and the hydrophilic segment 30b is formed in the space unit between the electrodes 15 and the hydrophobic segment 30a.

Meanwhile, the space film 20 is applied on the top surface of the electrodes 15, and the capillary tube 25 of a narrow width is added at the center of the outside of the space film 20 in the horizontal direction.

In addition, the blood cell separating pad 30 wider than the capillary tube 25 is attached on the top surface of the hydrophobic segment 30a and the hydrophilic segment 30b.

Then, manufacturing of the biosensor according to the present invention is completed by attaching the cover film 40 on the top surface of the capillary tube 25 and the blood cell separating pad 30.

FIG. 2 is a view showing another embodiment of the present invention, and it is a structure in which positions of the capillary tube 25 and the blood cell separating pad 30 added at one side of the biosensor 50 are changed.

That is, the blood cell separating pad 30 is attached at the outer side of the space film 20, and the capillary tube 25 is formed to be extended long in the horizontal direction at the outer side of the blood cell separating pad 30.

The hydrophobic segment 30a attached with a double-sided tape in the vertical direction and the hydrophilic segment 30b having an empty space are formed under the blood cell separating pad 30.

In addition, the cover film 40 is attached on the top surface of the blood cell separating pad 30 and the capillary tube 25, and a method of manufacturing a biosensor and effects obtained from the structure of the another embodiment are almost the same as those of the previous embodiment.

Operating states of the present invention configured as described above will be described below with reference to FIGs.2 (a) and 2(b).

That is, if blood sampled from a person taking a test is injected into the blood injection hole 33, the blood cell separating pad 30 absorbs the blood.

The blood absorbed into the blood cell separating pad 30 is separated into plasma and blood cells contained in the blood due to a difference in resistance while the blood passes through the hydrophobic segment 30a formed on the bottom surface.

After the blood is separated into the plasma and the blood cells, the plasma moves to the front side of the capillary tube 25 faster than the blood cells while the plasma and the blood cells pass through the hydrophilic segment 30b.

A certain amount of the moved plasma is absorbed into the capillary tube 25 owing to the capillary tube phenomenon.

A measurement is performed in a conventional electrochemical method or liquid color developing method using the absorbed plasma, and thus values resulting from an analysis are promptly and accurately obtained.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. A disease diagnosing biosensor capable of promptly separating blood cells, the biosensor comprising:
a base film;
electrodes arranged on a top surface of the base film;
a space film covering a top surface of the electrodes;
a blood cell separating pad connected to a rear side of the space film and connected to a capillary tube;
the capillary tube added on a rear top surface of the space film; and
a cover film covered on a top surface of the capillary tube and the blood cell separating pad.

2. The biosensor according to claim 1, wherein the blood cell separating pad is added to either a front side or a rear side of the capillary tube.

3. The biosensor according to claim 1, wherein the blood cell separating pad is manufactured using any one of cellulose, fusion 5, glass fiber, cotton, and wool.

4. The biosensor according to claim 1, wherein the blood cell separating pad is manufactured to have a width of 0.5 to 20mm.

5. The biosensor according to claim 1, wherein the biosensor is manufactured to have a hydrophobic segment on a bottom surface of the blood cell separating pad.

6. The biosensor according to claim 5, wherein the biosensor is manufactured by processing the hydrophobic segment formed on the bottom surface of the blood cell separating pad to have a width of 0.5 to 20mm, using a double-sided tape or a material having hydrophobic properties.

7. The biosensor according to claim 1, wherein the biosensor is manufactured to have a hydrophilic segment on a bottom surface of the blood cell separating pad.

8. The biosensor according to claim 5, wherein the biosensor is manufactured by processing the hydrophilic segment formed on the bottom surface of the blood cell separating pad to have a width of 0.5 to 20mm, using a surfactant agent or a material having hydrophilic properties.
